# EUROPEAN PATENT SPECIFICATION

(11) **EP 3 662 848 B1**
(45) Date of publication and mention of the grant of the patent: **05.07.2023**
(21) Application number: 18840326.5
(22) Date of filing: 16.07.2018
(51) Int. Cl.: A61B 17/22, A61B 17/3207, A61M 1/00

(54) **THROMBUS REMOVAL APPARATUS**
VORRICHTUNG ZUR ENTFERNUNG VON THROMBEN
APPAREIL D'ÉLIMINATION DE THROMBUS

(30) Priority: 01.08.2017 CN 201710648523
(43) Date of publication of application: 10.06.2020
(73) Proprietor: Shanghai Bluevascular Medtech Co., Ltd., Shanghai 201321 (CN)
(72) Inventor: HAN, Jianchao, Shanghai 201318 (CN); ZHANG, Linlin, Shanghai 201318 (CN); FAN, Yaming, Shanghai 201318 (CN); DING, Shuangxi, Shanghai 201318 (CN); SHI, Zengzuo, Shanghai 201318 (CN); LI, Zhonghua, Shanghai 201318 (CN); MIAO, Zhenghua, Shanghai 201318 (CN)
(74) Representative: Lavoix
(86) International application number: PCT/CN2018/095855
(87) International publication number: WO 2019/024678

(56) References cited:
- EP-A1- 3 141 201
- WO-A1-2009/005779
- WO-A1-2017/041062
- CN-A- 101 730 507
- CN-A- 102 319 097
- CN-A- 103 281 973
- CN-U- 205 126 342
- US-A1- 2004 219 028
- US-A1- 2006 259 052
- US-A1- 2014 088 610

## Description

### TECHNICAL FIELD

The present invention relates to the technical field of medical instruments and, more specifically, to a mechanical thrombectomy device.

### BACKGROUND

Deep Venous Thrombosis (DVT) refers to abnormal blood clotting in deep veins of lower extremities, which is a lower extremity venous return disorder disease. There are two main complications of DVT: no timely treatment is carried out in the early stage of thrombosis, which will cause Post Thrombosis Syndrome (PTS), characterized by lower extremity edema, muscle pain, chromatosis, subcutaneous tissue fiber changes, and even local ulcer. The thrombus detaches and reaches the pulmonary artery with blood shock, which can cause Pulmonary Embolism (PE). The diagnostic rate of PE is low, and the rate of misdiagnosis and mortality is high.

DVT can be treated with oral anticoagulants such as aspirin and warfarin at the early stage. This treatment can inhibit the formation and development of thrombosis and reduce the incidence of complications. However, there are also significant defects in drug treatment. For patients with prone to hemorrhage, severe liver and kidney disease, and high blood pressure, brain, spinal cord and ophthalmic surgery patients, pregnant women and the like, the drug treatment may cause bleeding, and even life-threatening in severe cases.

Catheter-Directed Thrombolysis (CDT) is another thrombosis treatment technique. Thrombolytic drugs such as urokinase and streptokinase are directly infused to the thrombosis site through a thrombolysis catheter to achieve the purpose of dissolving the thrombosis. CDT can restore blood circulation to some extent, release or relieve venous obstruction. However, the treatment efficiency is low and the treatment period is long. The thrombolysis catheter needs to be placed in the patient's body for a long time, which seriously affects the quality of life of the patient. In addition, the large use of thrombolytic drugs increases the risk of bleeding. Therefore, CDT is not suitable for patients with high bleeding risk and is not suitable for patients with pregnancy and childbirth.

For patients with severe symptoms and inability to use anticoagulants and thrombolytic drugs, conventional surgical methods can be used for treatment. However, this method can cause a large wound in the patient. After the operation, the patient suffers from greater pains, slow recovery, and long hospital stay. In addition, patients with poor general conditions are not suitable for conventional surgery.

Large lumen catheter aspiration therapy is to aspirate the thrombosis by the negative pressure provided by the syringe to achieve the purpose of thrombectomy. Such a treatment device is simple in structure and convenient to operate. During the operation, the surgeon can intuitively perceive the aspiration pressure in the blood vessel, and the thrombectomy process can be determined according to the pressure change. However, this treatment device has a great defect. When a massive thrombosis is inhaled in the catheter, the lumen is easily blocked, which seriously affects the efficiency of the thrombectomy.

With the development of technology, the Percutaneous Mechanical Thrombectomy (PMT) devices that have emerged in recent years are increasingly favored by surgeons and patients. PMT is a micro trauma interventional treatment method that effectively removes thrombosis by means of dissolution, fragmentation and aspiration to restore blood circulation.

The patent publications US2014/0088610A1 and WO2011/024124A1 respectively disclose a PMT device, the power sources of which both use electric energy. A negative pressure is formed in the catheter through physical transformation to aspirate the thrombus. The thrombus aspirated into the lumen is fragmentated into small thrombi and then transported out of the body to achieve the purpose of removing the thrombi. Both devices can effectively remove thrombi and restore blood circulation. However, the foregoing PMT device has the following disadvantages: 1) since the negative pressure fluctuation for aspirating the thrombus is small (substantially constant), it is difficult to aspirate the thrombus into the lumen when the thrombus outside the catheter adapts to such a pressure and the position is poor, resulting in more residual thrombi in the blood vessels and a low rate of thrombus removal; 2) the system automatically aspirates and breaks the thrombus from the blood vessels after the device is turned on, the surgeon cannot intuitively perceive whether the thrombus is aspirated, the process of thrombus removal and abnormal conditions; and 3) the active part of the device is complicated to operate, the surgeon needs to take care of both the machine and the patient during the operation, and cannot put more attention into the treatment of the patient.

WO 2017/041062 A1 describes a system for treating a patient having thrombus.

US 2006/259052 A1 describes a thrombectomy catheter.

US 2004/219028 A1 describes a mechanical pump for removal of fragmented matter.

WO 2009/005779 A1 describes devices and methods for the treatment of occluded body lumens, in particular for the removal of occluding material from blood vessels.

For the deficiencies of existing PMT devices, those skilled in the art have been searching for a solution.

### SUMMARY OF THE DISCLOSURE

Claim 1 defines the invention and dependent claims disclose embodiments. No surgical methods form part of the invention.

One of the objectives of the present invention is to solve the problem in the prior art that the massive thrombosis blocks the lumen, which affects the thrombectomy efficiency.

Another objective of the present invention is to solve the problem that the surgeon cannot accurately determine the surgical process when using electric-powered aspiration, thereby improving the surgical accuracy.

A further objective of the present invention is to solve the problem of complicated operation of the active part of the device.

In order to solve the foregoing technical problems, the present invention provides a mechanical thrombectomy device according to claim 1.

Optional features of the mechanical thrombectomy device are defined in claims 2 to 13.

In the mechanical thrombectomy device provided by the present invention, the thrombus aspiration system and the thrombus fragmentation system can be driven simultaneously by means of a manual operating assembly. Before thrombus extraction, the thrombus is already cut into small thrombi by means of the thrombus fragmentation system, avoiding the defect in the prior art of massive thrombi blocking the lumen, which affects the efficiency of thrombus removal, and improving the surgical success rate.

On the other hand, the manual operating assembly makes it possible to intuitively sense the aspiration pressure inside the blood vessel and, on the basis of the change in aspiration pressure, it is possible to intuitively perceive whether the thrombus is aspirated, the process of thrombus removal and abnormal conditions, avoiding the defect in the prior art that the surgeon cannot accurately determine the surgical process when using electric-powered aspiration, and improving the surgical accuracy.

On the other hand, when a pressing force is applied to the press handle, the thrombus aspirated by the thrombus removal head is transferred to the aspiration assembly through the sheath and the liquid inlet tube under the action of a negative pressure formed in the aspiration assembly. When the press handle is released from the pressing force, the thrombus that has been transferred into the aspiration assembly is transferred to a collection container through the drainage tube under the action of a positive pressure formed in the aspiration assembly. Since the thrombus aspiration process is completed by pressing the press handle, the thrombus aspiration process exhibits a fluctuant aspiration, and the fluctuant negative pressure is used to effectively aspirate the poorly located thrombus into the lumen and take out of the patient's body. Moreover, the device is simple to operate, and thrombectomy can be implemented by only pressing the press handle, thereby further improving the thrombus removal rate.

On the other hand, the present invention also provides a mechanical thrombectomy device integrating perfusion of thrombolytic drugs, negative pressure aspiration thrombectomy, and mechanical thrombus fragmentation, which can significantly accelerate the thrombectomy process, shorten the operation time, alleviate the pain of patients, and expand the scope of application.

### BRIEF DESCRIPTION OF THE DRAWINGS

FIG. 1 is a schematic structural diagram of a mechanical thrombectomy device according to Embodiment 1 of the present invention;
FIG. 2 is a schematic structural diagram of a thrombus aspiration system according to Embodiment 1 of the present invention;
FIG. 3 is a schematic cross-sectional view of an aspiration assembly of FIG. 2;
FIG. 4 is a schematic structural diagram of a thrombus fragmentation system according to Embodiment 1 of the present invention;
FIG. 5 is a partial cross-sectional view of a thrombus removal head of Embodiment 1 of the present invention;
FIG. 6 is a cross-sectional view of a sheath connector of Embodiment 1 of the present invention;
FIG. 7 is a schematic structural diagram of a mechanical thrombectomy device according to Embodiment 2 of the present invention; and
FIG. 8 is a schematic structural diagram of a gear assembly according to Embodiment 1 of the present invention.

### In the drawings:

100-mechanical thrombectomy device; 1-thrombus aspiration system; 1000-operating member; 1110-press handle; 1130-shell; 1120-torsion spring; 1310-connecting member; 1320-aspiration assembly; 1321-aspiration chamber; 1322-liquid inlet chamber; 1323-drainage chamber; 1327-hinge pin; 1326-piston; 1325-piston rod; 1324-first check valve; 1324'-second check valve; 1370-drainage tube; 1380-collection container; 1360-liquid inlet tube; 1340-sheath; 1331-top hole; 1332-side hole; 1333-fixing ring; 2-thrombolytic system; 1410-syringe; 1420-third check valve; 1430-infusion tube; 3-thrombus fragmentation system; 1210-transmission member; 1200-gear assembly; 1220-first gear; 1230-second gear; 1223-ratchet; 1240-first bevel gear; 1250-second bevel gear; 1241-third gear; 1260-transmission tube; 1263-front transmission tube; 1262-intermediate transmission tube; 1262a-side hole; 1261-rear transmission tube; 1280-transmission tube end connector; 1270-thrombus fragmentation cutter; 1271-thrombus fragmentation cutter blade; 4-thrombus removal head; 1350-sheath connector; 1350a-main connector; 1350b-first branch connector; 1350c-second branch connector; 1350d-third branch connector; 1231-gear plate; 1311-connecting rod; 1312-sliding block.

### DETAILED DESCRIPTION

The mechanical thrombectomy device proposed by the present invention will be further described in detail below with reference to the accompanying drawings and specific embodiments. Advantages and features of the present invention will be apparent from the description and appended claims below. It should be noted that the drawings are in a very simplified form and use non-precise proportions, and are only intended to conveniently and explicitly assist in describing the objectives of embodiments of the present invention.

As used herein, a "proximal end" and a "distal end" are defined as follows: the "distal end" generally refers to the end of a medical device that first enters the patient during normal operation, while the "proximal end" generally refers to the end of the medical device close to the operator during the normal operation.

### Embodiment 1

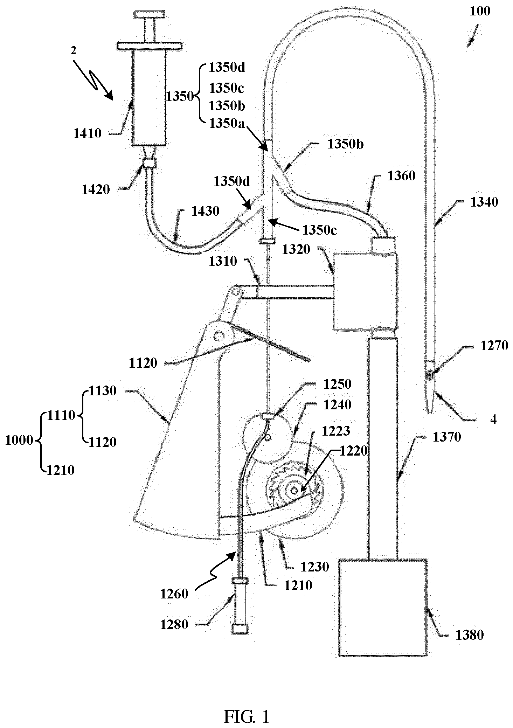

Referring to FIG. 1, it is a schematic structural diagram of a mechanical thrombectomy device according to this embodiment. As shown in FIG. 1, the mechanical thrombectomy device 100 includes a thrombus removal head 4, a thrombus fragmentation system 3, and a thrombus aspiration system 1. The thrombus removal head 4 is connected to a sheath 1340.

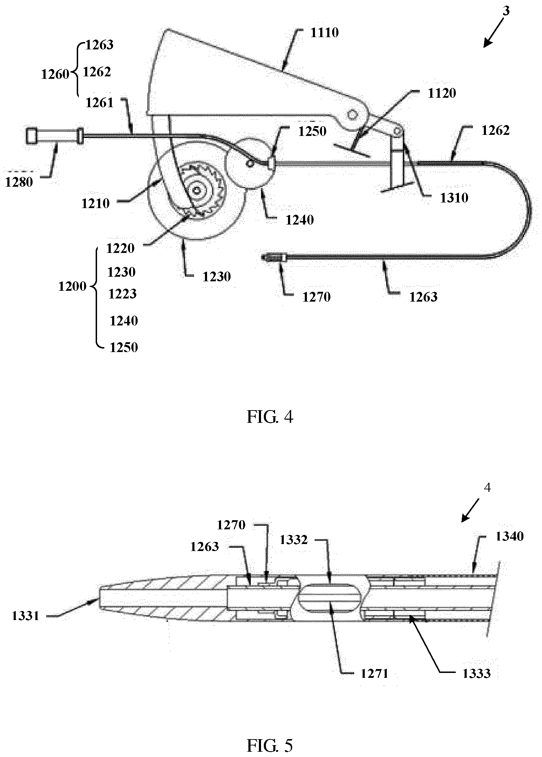

Referring to FIG. 4 mainly, it is a schematic structural diagram of the thrombus fragmentation system 3 according to this embodiment. As shown in FIG. 4, the thrombus fragmentation system 3 includes an operating assembly 1000, a gear assembly 1200, a transmission tube 1260, and a thrombus fragmentation cutter 1270. The operating assembly drives the transmission tube 1260 to rotate through the gear assembly 1200, and a distal end of the transmission tube 1260 is connected to the thrombus fragmentation cutter 1270. The operating assembly 1000 includes, for example, a press handle 1110 and a transmission member 1210. The transmission member 1210 transmits the acting force on the press handle 1110 to the gear assembly 1200.

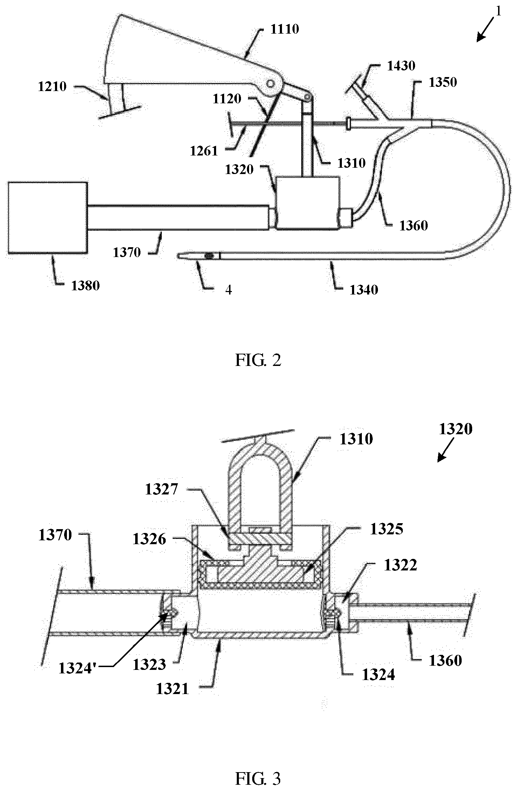

Referring to FIG. 2 mainly, the thrombus aspiration system 1 comprises a connecting member 1310, an aspiration assembly 1320, a drainage tube 1370, and a liquid inlet tube 1360. One end of the connecting member 1310 is connected to and linked with the operating assembly, and the other end of the connecting member 1310 is connected to the aspiration assembly 1320. The connecting member 1310 drives the aspiration assembly 1320 to form a pressure chamber. The pressure chamber is connected to the drainage tube 1370 and the liquid inlet tube 1360. The liquid inlet tube 1360 is connected to the sheath 1340.

Still referring to FIG. 2, one end of the drainage tube 1370 is communicated with the aspiration assembly 1320, and the other end of the drainage tube 1370 is communicated with a collection container 1380. One end of the liquid inlet tube 1360 is communicated with the aspiration assembly 1320, and the other end of the liquid inlet tube 1360 is connected to one end of the sheath 1340, and the other end of the sheath 1340 is connected to the thrombus removal head 4. The collection container 1380 is preferably a collection bag or collection bin.

Referring to FIG. 5, it is a partial cross-sectional view of the thrombus removal head 4 according to this embodiment. The thrombus removal head 4 is connected to the sheath 1340 through a fixing ring 1333. The thrombus removal head 4 has a top hole 1331 configured to convey a thrombolytic drug and a side hole 1332 for aspirating the thrombus. To facilitate the fragmentation of the thrombus, the location of the side hole 1332 corresponds to the location of a thrombus fragmentation cutter 1270 such that the thrombus aspirated in the thrombus removal head 4 through the side hole 1332 is directly fragmentated by a thrombus fragmentation cutter blade 1271 of the thrombus fragmentation cutter.

In order to meet the function of the aspiration assembly, as shown in FIG. 3, the aspiration assembly 1320 includes an aspiration chamber 1321, a piston 1326, and a piston rod 1325. The piston 1326 sleeves over the piston rod 1325 and is tangent with an inner surface of the aspiration chamber 1321. One end of the piston rod 1325 away from the piston 1326 is connected to the connecting member 1310. A negative pressure is generated in the pressure chamber when a force is applied to the operating assembly, and a positive pressure is generated in the pressure chamber when no force is applied to the operating assembly. Specifically, when a force is applied to the operating assembly (specifically, when a pressing force is applied to the press handle 1110), the press handle 1110 sequentially drives the connecting member 1310, the piston rod 1325, and the piston 1326 to move along a direction which allows disengagement from the aspiration chamber 1321, so as to form a negative pressure in the aspiration chamber 1321. When the press handle 1110 is released from the pressing force, the press handle 1110 automatically returns to the natural state under the action of the torsion spring 1120, so as to sequentially drive the connecting member 1310, the piston rod 1325, and the piston 1326 to move along the direction which allows entry into the aspiration chamber 1321, to form a positive pressure in the aspiration chamber 1321. The structure of the aspiration assembly 1320 includes, but is not limited to, the structure shown in FIG. 3, as long as the aspiration assembly 1320 can change the internal pressure of the aspiration chamber 1321 under the linkage action of the pressing force to the press handle 1110, thereby forming a negative pressure in the aspiration assembly 1320 to meet the pressure requirements for aspiration of the thrombus.

Further, when the aspiration assembly 1320 is in operation, either a state for aspiration or a state for drainage is available at every moment. In order to control the flow direction of the liquid in the aspiration assembly 1320 in the case that the aspiration assembly 1320 is communicated with both the drainage tube 1370 and the liquid inlet tube 1360, the aspiration assembly 1320 further includes: a first check valve 1324 and a second check valve 1324'. The first check valve 1324 is disposed in the liquid inlet tube 1360, and the second check valve 1324' is disposed in the drainage tube 1370. When a negative pressure is formed in the aspiration assembly 1320, under the action of the negative pressure, the first check valve 1324 is open, the second check valve 1324' is closed, and the thrombus aspirated by the thrombus removal head 4 is transferred into the aspiration chamber 1321 through the sheath 1340 and the liquid inlet tube 1360 (in this case, the aspiration assembly 1320 is in an aspiration state). When a positive pressure is formed in the aspiration assembly 1320, under the action of the positive pressure, the first check valve 1324 is closed, the second check valve 1324' is open, and the thrombus that has been transferred into the aspiration chamber 1321 is transferred to a collection container 1380 through the drainage tube 1370 (in this case, the aspiration assembly 1320 is in a drainage state).

Preferably, the first check valve 1324 and the second check valve 1324' are both umbrella valves. The aspiration chamber 1321 is of a shell structure and has a liquid inlet chamber 1322 and a drainage chamber 1323. The liquid inlet chamber 1322 is communicated with the liquid inlet tube 1360 (the liquid inlet tube 1360 can be adhered to the outer side of the liquid inlet chamber 1322 by glue bonding), and the drainage chamber 1323 is communicated with the drainage tube 1370 (the drainage tube 1370 can be adhered to the outer side of the drainage chamber 1323 by glue bonding), and the liquid inlet chamber 1322 and the drainage chamber 1323 each have a flange. The umbrella valves are mounted on the corresponding flanges, and the umbrella valve in the liquid inlet chamber 1322 and the umbrella valve in the drainage chamber 1323 are disposed in the same direction. Therefore, the umbrella valve in the drainage chamber 1323 is automatically closed under the internal pressure of the aspiration chamber when the umbrella valve in the liquid inlet chamber 1322 is open, and vice versa.

In this embodiment, the connecting member 1310 may have two specific structures, as follows.

Structure 1: the connecting member 1310 is a connecting rod, one end of the connecting rod is connected to the press handle 1110 through a hinge pin, and the other end of the connecting rod passes through the body wall of the aspiration chamber 1321 and then is connected to the piston rod 1325. When a pressing force is applied to the press handle 1110, the connecting rod drives the piston rod 1325 to move.

Structure 1 has the advantage of simple structure, but may have a disadvantage as follows. Since the connecting rod is a rod-shaped body, the connecting rod drives the piston rod 1325 connected thereto to cause a displacement in both the horizontal direction and the vertical direction under the drive of the acting force on the press handle 1110. Therefore, there is a relative horizontal movement between the piston 1326 and the aspiration chamber 1321, which is detrimental to the sealing effect of the piston 1326 in the aspiration chamber 1321, increasing abrasion of the piston 1326 and the aspiration chamber 1321.

Structure 2: referring to FIG. 3, the connecting member 1310 is of a U-shaped structure, and a bottom connecting rod of the U-shaped structure is connected to the press handle 1110, and an open end of the U-shaped structure is connected to the aspiration assembly 1320 (specifically, the open end extends through the body wall of the aspiration chamber 1321 and then is connected to the piston rod 1325 through the hinge pin 1327). Structure 2 overcomes the possible problem of structure 1. The open end of the U-shaped structure extends through the body wall of the aspiration chamber 1321 and then is connected to the piston rod 1325 through the hinge pin 1327, so that the piston rod 1325 can be move horizontally along the hinge pin 1327 traversing the open end of the U-shaped structure, and thus the piston 1326 does not cause a horizontal displacement relative to the aspiration chamber 1321, avoiding a rubbing between the piston 1326 and the body wall of the aspiration chamber 1321. In the technical solution, by using the U-shaped structure, the piston rod is not subjected to the torsion during movement, and the direction of the acting force applied to the piston rod is always on the axis of the aspiration chamber.

Still referring to FIG. 1, the press handle 1110 includes a shell 1130 and a torsion spring 1120 mounted in the shell 1130 through a hinge pin. When the press handle 1110 is released from the pressing force, the press handle 1110 automatically returns to the natural state under the action of the torsion spring 1120.

In this embodiment, as shown in FIG. 1, the mechanical thrombectomy device may further include a thrombolytic system 2. The thrombolytic system 2 includes a syringe 1410, an infusion tube 1430, and a third check valve 1420. The infusion tube 1430 is connected to the syringe 1410, and the third check valve 1420 is disposed in the infusion tube 1430. The syringe 1410 injects a thrombolytic drug loaded therein to a thrombosis site through the infusion tube 1430 to soften or dissolve the thrombus of the thrombosis site. The purpose of the third check valve 1420 is to prevent backflow of the thrombolytic drug in the infusion tube 1430, thereby achieving one-way flow of the thrombolytic drug from the syringe 1410 to the infusion tube 1430.

Further, the thrombus fragmentation system 3 further includes a transmission tube end connector 1280 and an adjustable hemostatic valve (not shown in FIG. 4) disposed on the transmission tube end connector 1280.

Referring to FIGs. 1 and 4, one end of the transmission member 1210 is fixedly connected to the press handle 1110, and the other end of the transmission member 1210 is meshed with the gear assembly 1200. One end of the transmission tube 1260 is connected to the transmission tube end connector 1280, and the other end (distal end) of the transmission tube 1260 is fixedly connected to the thrombus fragmentation cutter 1270 and received in the sheath 1340. The transmission member 1210 transfers the acting force from the press handle 1110 to the gear assembly 1200, and the gear assembly 1200 transfers a rotational motion to the transmission tube 1260 under the acting force from the press handle 1110. The transmission tube 1260 drives the thrombus fragmentation cutter 1270 to rotate, so as to fragmentate the thrombus that has been aspirated into the thrombus removal head 4. In this embodiment, the transmission member 1210 is preferably a rack, and more preferably an arc rack.

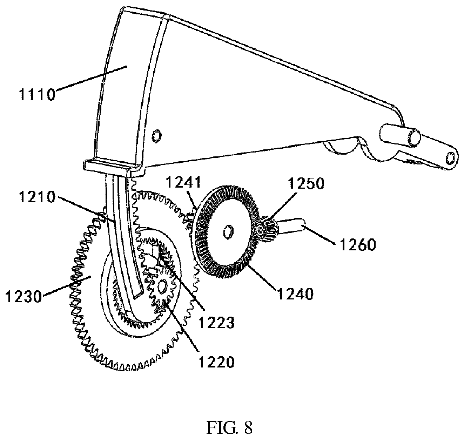

As shown in FIG. 8, the gear assembly 1200 includes a first bevel gear 1240 and a second bevel gear 1250. The first bevel gear 1240 is meshed with the second bevel gear 1250, and the second bevel gear 1250 is fixed to the transmission tube 1260. Further, the gear assembly further includes a first gear 1220, a second gear 1230, a ratchet 1223, and a third gear 1241. The transmission member 1210 is meshed with the first gear 1220, the first gear 1220 shares the ratchet 1223 with the second gear 1230, the second gear 1230 is meshed with the third gear 1241, the third gear 1241 is coaxial with the first bevel gear 1240, and the second bevel gear 1250 is meshed with the first bevel gear 1240.

Referring to FIG. 4 or 8, the working process of the gear assembly 1200 is as follows:
When a pressing force is applied to the press handle 1110, the transmission member 1210 fixedly connected to the press handle 1110 moves to the lower right of the drawing, the thrombus fragmentation transmission member 1210 drives the first gear 1220 to rotate counterclockwise, the first gear 1220 drives the second gear 1230 to rotate counterclockwise, the second gear 1230 drives the first bevel gear 1240 to rotate through the third gear, the first bevel gear 1240 drives the second bevel gear 1250 to rotate, the second bevel gear 1250 drives the transmission tube 1260 to rotate, and the transmission tube 1260 drives the thrombus fragmentation cutter 1270 to rotate.

When the press handle 1110 is released from the pressing force, the transmission member 1210 fixedly connected to the press handle 1110 moves to the upper left of the drawing, and the transmission member 1210 drives the first gear 1220 to rotate clockwise. Under the action of the ratchet 1223, the second gear 1230 does not rotate with the rotation of the first gear 1220, that is, the transmission of the rotation of the first gear 1220 is stopped at the ratchet 1223, and the second gear 1230, the first bevel gear 1240, the second bevel gear 1250, and the transmission tube 1260 do not rotate.

As shown in FIG. 1, the thrombus fragmentation system 4 and the thrombus aspiration system 1 are both connected to the sheath 1340 through the sheath connector 1350, and the transmission tube 1260 extends through the sheath connector 1350 and is received in the sheath 1340.

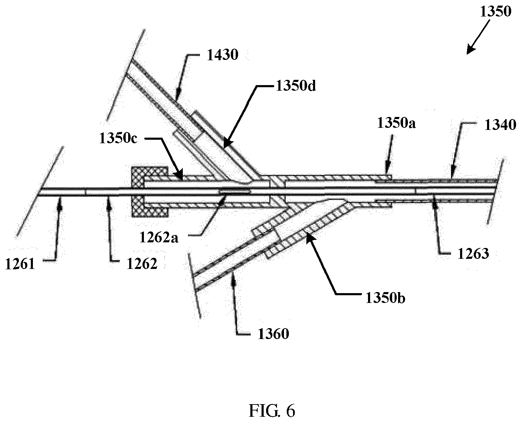

One end of the sheath connector 1350 is connected to one end of the sheath 1340 away from the thrombus removal head 4. The connection between the sheath 1340 and the thrombus aspiration system 1, the connection between the sheath 1340 and the thrombolytic system 2, and the connection between the sheath 1340 and the thrombus fragmentation system 3 are achieved by the sheath connector 1350. Specifically, the transmission tube 1260 includes: a front transmission tube 1263, an intermediate transmission tube 1262, and a rear transmission tube 1261 that are sequentially arranged and communicated with each other. The front transmission tube 1263 and the rear transmission tube 1261 are hollow tubular members of single-layer or multi-layer composite tubes. The front transmission tube 1263 and the rear transmission tube 1261 can include a metal braided layer and a liner layer in the case of the multi-layer composite tubes. The intermediate transmission tube 1262 has at least one side hole 1262a (see FIG. 6), and the liquid entering the sheath connector 1350 enters the intermediate transmission tube 1262 through the side hole 1262a. The thrombus fragmentation cutter 1270 is fixed to the front transmission tube 1263 and is received in the sheath 1340. The intermediate transmission tube 1262 is received in the sheath connector 1350. One end of the rear transmission tube 1261 away from the intermediate transmission tube 1262 is connected to the transmission tube end connector 1280, and the second bevel gear 1250 sleeves over the rear transmission tube 1261 (in other words, the rear transmission tube 1261 extends through the center hole of the second bevel gear 1250 and is fixedly connected to the second bevel gear 1250, so that the rear transmission tube 1261 rotates with the second bevel gear 1250 when the second bevel gear 1250 rotates, thereby driving the thrombus fragmentation cutter 1270 fixed on the front transmission tube 1263 to rotate for thrombus fragmentation).

In order to achieve the connection between the sheath 1340 and the thrombus aspiration system 1, the connection between the sheath 1340 and the thrombolytic system 2, and the connection between the sheath 1340 and the thrombus fragmentation system 3, as shown in FIG. 6, the sheath connector 1350 is designed to include: a main connector 1350a, a second branch connector 1350c and a third branch connector 1350d that are communicated with one end of the main connector 1350a, and a first branch connector 1350b that is communicated with the other end of the main connector 1350a, wherein the other end of the main connector 1350a is also communicated with the sheath 1340. In the thrombus aspiration system 1, the liquid inlet tube 1360 is connected to the sheath 1340 through the first branch connector 1350b, and the thrombus fragmentation system 3 is connected to the sheath 1340 through the second branch connector 1350c. In the thrombolytic system 2, the infusion tube 1430 is communicated with the sheath 1340 through the third branch connector 1350d, and the liquid (e.g., the thrombolytic drug) conveyed to the sheath 1340 through the infusion tube 1430 can enter the transmission tube through the side hole 1262a on the intermediate transmission tube 1262.

In the preparation of various members of the mechanical thrombectomy device of this embodiment, each member can be fabricated by machining, injection molding, extrusion, laser engraving and other processing modes. The fixed connection between different members can be done by laser welding or glue bonding. For example, the front transmission tube 1263, the intermediate transmission tube 1262, and the rear transmission tube 1261 are sequentially welded by laser to form a transmission tube, and the thrombus fragmentation cutter 1270 is fixed to the front transmission tube 1263 by laser welding. Since the preparation and assembly modes of the members are not the focus of the technical solution of the present invention, no further details are provided herein.

In order to better understand the mechanical thrombectomy device of the present invention, the application process, not forming part of the present invention, of the mechanical thrombectomy device will be described in detail below with reference to FIG. 1, and the specific process is as follows:
Before the operation, the thrombus removal head 4 is placed in normal saline, and the press handle 1110 is manually pressed until the normal saline is visible in the collection container 1380. The syringe 1410 is connected to the third check valve 1420 for aspiration. The aspiration is stopped when the normal saline is visible in the syringe 1410, and the air in the mechanical thrombectomy device is evacuated. The action principle of the aspiration assembly 1320 when the press handle 1110 is manually pressed before the operation is the same as that when the press handle 1110 is manually pressed during the operation.

During the operation, the blood vessel is punctured, and the mechanical thrombectomy device reaches the thrombosis site along the guide wire (outsourced). Specifically, both the sheath 1340 and the thrombus removal head 4 enter the patient's body, and the remaining structures are located outside the patient's body, and the thrombus removal head 4 reaches the thrombosis site. The thrombolytic process: after aspirating the thrombolytic drug, the syringe 1410 is connected and communicated with the third check valve 1420, the syringe 1410 is pressed to sequentially inject the thrombolytic drug into an inner cavity of the intermediate transmission tube 1262 and an inner cavity of the front transmission tube 1263 through the infusion tube 1430 and the side hole 1262a on the intermediate transmission tube 1262, and the thrombolytic drug finally reaches the thrombosis site through a top hole 1331 of the thrombus removal head 4, and the thrombolytic drug is in contact with the thrombus to soften or dissolve the thrombus, so as to complete the thrombolytic process. Thrombus aspiration and thrombus fragmentation are carried out after a predetermined period of time. The press handle 1110 is pressed, the gear assembly 1200 transmit the rotational motion to the transmission tube under the action of the press handle 1110, and then the transmission tube drives the thrombus fragmentation cutter 1270 to rotate; and meanwhile, the press handle 1110 drives the piston 1326 to aspirate and discharge in the aspiration chamber 1321 through the connecting member 1310 and the piston rod 1325. When the press handle 1110 is manually pressed, the piston 1326 moves outward from the aspiration chamber 1321, and the aspiration assembly 1320 performs aspiration. When the press handle 1110 is released, the piston 1326 moves into the aspiration chamber 1321, and the aspiration assembly 1320 performs drainage. Since the umbrella valve in the liquid inlet chamber 1322 is disposed in the same direction as the umbrella valve in the drainage chamber 1323, the umbrella valve in the drainage chamber 1323 is automatically closed under the internal pressure of the aspiration chamber when the umbrella valve in the liquid inlet chamber 1322 is open, and vice versa. During aspiration, the umbrella valve in the liquid inlet chamber 1322 is open, the umbrella valve in the drainage chamber 1323 is closed, and a passage formed by the aspiration chamber 1321, the liquid inlet chamber 1322, the liquid inlet tube 1360, and the sheath 1340 aspirates the thrombus into the thrombus removal head 4 through the side hole 1332 of the thrombus removal head 4 under the negative pressure, and the rotating thrombus fragmentation cutter blade 1271 and the side hole 1332 of the thrombus removal head 4 cooperate to shred the aspirated thrombus, and meanwhile, the shredded thrombus is aspirated into the aspiration chamber 1321 under the negative pressure. During drainage, the umbrella valve in the liquid inlet chamber 1322 is closed, the umbrella valve in the drainage chamber 1323 is open, and a passage formed by the aspiration chamber 1321, the drainage chamber 1323, and the drainage tube 1370 discharges the shredded thrombus out of the patient's body under the positive pressure and the shredded thrombus is conveyed to the collection container 1380.

Upon completion of thrombectomy, the pressing of the press handle 1110 is stopped, and all the devices are withdrawn from the patient's body.

### Embodiment 2

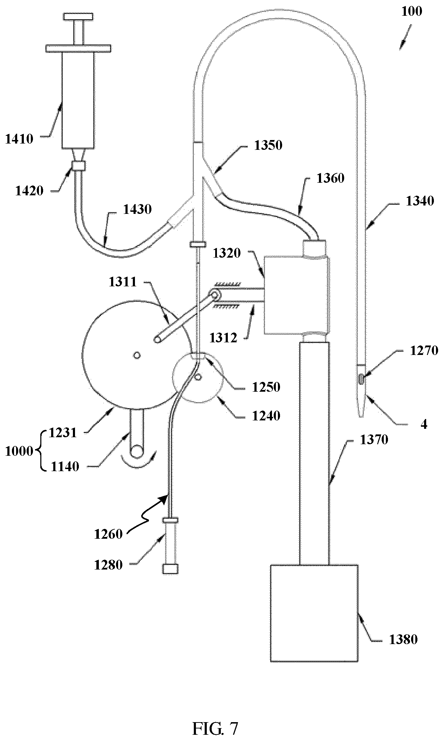

As shown in FIG. 7, this embodiment differs from Embodiment 1 in that the operating assembly 1000 includes a rocker 1140 and a gear plate 1231. The rocker 1140 is located at one side of the gear plate 1231 and is connected to the gear plate 1231 through a shaft. The gear plate 1231 is meshed with the third gear 1241. The third gear 1241 is coaxial with the first bevel gear 1240. The first bevel gear 1240 is meshed with the second bevel gear 1250.

In the preparation of various members of the mechanical thrombectomy device of this embodiment, each member can be fabricated by machining, injection molding, extrusion, laser engraving and other processing modes. The fixed connection between different members can be done by laser welding or glue bonding. For example, the front transmission tube 1263, the intermediate transmission tube 1262, and the rear transmission tube 1261 are sequentially welded by laser to form the transmission tube 1260.

As shown in FIGs. 2, 5, and 6, the proximal end of the intermediate transmission tube 1262 is welded to the rear transmission tube 1261, and the intermediate transmission tube 1262 is placed in the sheath connector 1350. One end of the rear transmission tube 1261 extends through the hole in the second bevel gear 1250 and the rear transmission tube 1261 is connected to the second bevel gear 1250 by laser welding, and the other end of the rear transmission tube 1261 is mounted in the transmission tube end connector 1280. The thrombus fragmentation cutter 1270 is fixed to the front transmission tube 1263 by laser welding and placed in the sheath 1340.

As shown in FIGs. 3 and 5-7, in this embodiment, the connecting rod 1311 is hinged with the gear plate 1231 at the other side of the gear plate 1231. One end of the sliding block 1312 is hinged with the connecting rod 1311, and the other end is connected to the piston rod 1325 through the hinge pin 1327. The first check valves 1324, 1324' are respectively mounted on the flanges in the liquid inlet chamber 1322 and the drainage chamber 1323, and are kept in the same direction. One end of the drainage tube 1370 is mounted at the outer end of the drainage chamber 1323 through glue bonding, and the other end is mounted to the collection container 1380. One end of the liquid inlet tube 1360 is mounted at the outer end of the liquid inlet chamber 1322 through glue bonding, and the other end is mounted to the first branch connector 1350b of the sheath connector 1350. The proximal end of the thrombus removal head 4 is welded to one end of the fixing ring 1333 through laser. The distal end of the sheath 1340 is bonded to the other end of the fixing ring 1333 by glue, and the proximal end of the sheath 1340 is bonded to the sheath connector 1350 by glue.

Referring to FIG. 7 mainly, one end of the infusion tube 1430 is bonded to the third branch connector 1350d of the sheath connector 1350 by glue, the third check valve 1420 is bonded to the other end of the infusion tube 1430 by glue, and the syringe 1410 is fixed to the third check valve 1420 to form a complete mechanical thrombectomy device 100.

In order to better understand the mechanical thrombectomy device of this embodiment, the application process, not forming part of the invention, of the mechanical thrombectomy device will be described in detail below with reference to FIGs. 3 and 5-7, and the specific process is as follows:
Before the operation, the thrombus removal head 4 is placed in normal saline, and the rocker 1140 is rotated counterclockwise until the normal saline is visible in the collection container 1380. The syringe 1410 is connected to the check valve 1420 for aspiration. The aspiration is stopped when the normal saline is visible in the syringe 1410, and the air in the mechanical thrombectomy device is evacuated. The action principle of the aspiration assembly 1320 when the rocker 1140 is rotated before the operation is the same as that when the rocker 1140 is rotated during the operation.

During the operation, the blood vessel is punctured, and the thrombectomy device reaches the thrombosis site along the guide wire (outsourced). Specifically, both the sheath 1340 and the thrombus removal head 4 enter the patient's body, and the remaining structures are located outside the patient's body. After aspirating the thrombolytic drug, the syringe 1410 is connected and communicated with the check valve 1420, and the syringe 1410 is pressed to sequentially inject the thrombolytic drug into the inner cavity of the intermediate transmission tube 1262 and the inner cavity of the front transmission tube 1263 through the infusion tube 1430 and the side hole 1262a on the intermediate transmission tube, and the thrombolytic drug finally reaches the thrombosis site through the top hole 1331, and the thrombolytic drug is in contact with the thrombus to soften or dissolve the thrombus. After a predetermined period of time, the rocker 1140 is rotated counterclockwise, and the gear system composed of the gear plate 1231, the third gear 1241, the first bevel gear 1240 and the second bevel gear 1250 transmit the rotational motion to the transmission tube 1260 under the action of the rocker 1140, and the transmission tube 1260 then drives the thrombus fragmentation cutter 1270 to rotate. Meanwhile, the rocker 1140 drives the piston 1326 to aspirate and discharge in the aspiration chamber 1321 through the gear plate 1231, the connecting rod 1311, the sliding block 1312 and the piston rod 1325. When the rocker 1140 rotates for the first half turn counterclockwise, the piston 1326 moves outward from the aspiration chamber 1321, and the aspiration assembly 1320 performs aspiration. When the rocker 1140 rotates for the second half turn counterclockwise, the piston 1326 moves into the aspiration chamber 1321, and the aspiration assembly 1320 performs drainage. Since the check valve in the liquid inlet chamber 1322 is disposed in the same direction as the check valve in the drainage chamber 1323, the check valve in the drainage chamber 1323 is automatically closed under the internal pressure of the aspiration chamber when the check valve in the liquid inlet chamber 1322 is open, and vice versa. During aspiration, the check valve in the liquid inlet chamber 1322 is open, the check valve in the drainage chamber 1323 is closed, and a passage formed by the aspiration chamber 1331, the liquid inlet chamber 1322, the liquid inlet tube 1360, and the sheath 1340 aspirates the thrombus into the thrombus removal head 4 through the side hole 1332 of the thrombus removal head under the negative pressure, and the rotating thrombus fragmentation cutter blade 1271 and the side hole 1332 of the thrombus removal head cooperate to shred the aspirated thrombus, and meanwhile, the shredded thrombus is aspirated into the aspiration chamber 1331 under the negative pressure. During drainage, the check valve in the liquid inlet chamber 1322 is closed, the check valve in the drainage chamber 1323 is open, and a passage formed by the aspiration chamber 1331, the drainage chamber 1323, and the drainage tube 1370 discharges the shredded thrombus out of the patient's body under the positive pressure and the shredded thrombus is conveyed to the collection container 1380.

Upon completion of thrombectomy, the rotation of the rocker 1140 is stopped, and all the devices are withdrawn from the patient's body.

In summary, the mechanical thrombectomy device of the present invention has the following beneficial effects:
(1) the integration of thrombus fragmentation, thrombolysis and thrombus avoids blockage of the lumen by massive thrombus and thus the efficiency of thrombectomy will not be affected;
(2) since the power of the thrombus aspiration and the thrombus disruption comes from the operation of the operator on the press handle or the rocker, the thrombus aspiration process exhibits a fluctuant aspiration, and the fluctuant negative pressure is used to effectively aspirate the poorly located thrombus into the lumen and take out of the patient's body, thereby further improving the thrombus removal rate;
(3) the aspiration pressure in the blood vessel can be sensed by manually pressing the press handle or swinging the rocker, and whether the thrombus is aspirated, the process of thrombus removal and abnormal conditions can be intuitively perceived according to the change in the pressure; and
(4) the device is easy to operate, and thrombectomy can be achieved only by pressing the press handle or swinging the rocker, so that the surgeon can put more attention into the treatment of the patient.

The above description is only for the description of preferred embodiments of the present invention, and is not intended to limit the scope of the present invention. The scope of the invention is defined by the appended claims.

## Claims

1. A thrombectomy device (100), **characterized in that** the thrombectomy device comprises:
a thrombus removal head (4) having at least one opening configured to allow thrombus to enter the thrombus removal head (4), and the thrombus removal head (4) being connected to a sheath (1340);
a thrombus fragmentation system (3) comprising an operating assembly, a gear assembly (1200), a transmission tube (1260) and a thrombus fragmentation cutter (1270), wherein the operating assembly drives the transmission tube (1260) to rotate through the gear assembly (1200), and a distal end of the transmission tube (1260) is connected to the thrombus fragmentation cutter (1270); and
a thrombus aspiration system (1) comprising a connecting member (1310), an aspiration assembly (1320), a drainage tube (1370) and a liquid inlet tube (1360), wherein one end of the connecting member (1310) is connected to the operating assembly, and a further end of the connecting member (1310) is connected to the aspiration assembly (1320); when driven by the operating assembly, the connecting member (1310) drives the aspiration assembly (1320) to form a pressure chamber; the pressure chamber is connected to the drainage tube (1370) and the liquid inlet tube (1360); and the liquid inlet tube (1360) is connected to the sheath (1340),
wherein the aspiration assembly (1320) comprises an aspiration chamber (1321), a piston (1326), and a piston rod (1325); the piston (1326) sleeves over the piston rod (1325) and is tangential with an inner surface of the aspiration chamber (1321); one end of the piston rod (1325) away from the piston (1326) is connected to the connecting member (1310), and wherein a negative pressure is generated in the pressure chamber when an acting force is applied to the operating assembly, and a positive pressure is generated in the pressure chamber when no acting force is applied to the operating assembly.

2. The thrombectomy device (100) according to claim 1, wherein the operating assembly comprises a press handle (1110) and a transmission member (1210); and the transmission member (1210) transmits an acting force from the press handle (1110) to the gear assembly (1200).

3. The thrombectomy device (100) according to claim 2, wherein the press handle (1110) comprises a shell (1130) and a torsion spring (1120) mounted in the shell (1130) through a hinge pin (1327); when the press handle (1110) is released from a pressing force, the press handle (1110) automatically returns to a natural state under an action of the torsion spring (1120).

4. The thrombectomy device (100) according to claim 2, wherein the transmission member (1210) is an arc rack.

5. The thrombectomy device (100) according to claim 1, wherein the operating assembly comprises a rocker and a gear plate (1231), and the gear plate (1231) is meshed with the gear assembly (1200).

6. The thrombectomy device (100) according to claim 1, wherein the gear assembly (1200) comprises a first bevel gear (1240) and a second bevel gear (1250); the first bevel gear (1240) is meshed with the second bevel gear (1250); the second bevel gear (1250) is fixed to the transmission tube (1260).

7. The thrombectomy device (100) according to claim 6, wherein the gear assembly (1200) further comprises a first gear (1220), a second gear (1230), a ratchet (1223), and a third gear (1241); the operating assembly is meshed with the first gear (1220); the first gear (1220) shares the ratchet (1223) with the second gear (1230); the second gear (1230) is meshed with the third gear (1241); and the third gear (1241) is coaxial with the first bevel gear (1240).

8. The thrombectomy device (100) according to any one of claims 1 to 7, wherein the aspiration assembly (1320) further comprises a first check valve (1324) and a second check valve (1324'); the first check valve (1324) is disposed in the liquid inlet tube (1360), and the second check valve (1324') is disposed in the drainage tube (1370).

9. The thrombectomy device (100) according to claim 8, wherein the first check valve (1324) and the second check valve (1324') are disposed in a same direction such that one of the first check valve (1324) and the second check valve (1324') is open while the other is closed.

10. The thrombectomy device (100) according to claim 1, wherein the connecting member (1310) is of a U-shaped structure; a bottom connecting rod (1311) of the U-shaped structure is connected to the operating assembly, and an opening end of the U-shaped structure is connected to the aspiration assembly (1320).

11. The thrombectomy device (100) according to claim 1, wherein the thrombus removal head (4) comprises a sheath connector (1350); the thrombus fragmentation system (3) and the thrombus aspiration system (1) are connected to the sheath (1340) through the sheath connector (1350); and the transmission tube (1260) extends through the sheath connector (1350) and is received in the sheath (1340).

12. The thrombectomy device (100) according to claim 1, wherein the thrombus fragmentation system (3) further comprises a transmission tube end connector (1280) and an adjustable hemostatic valve disposed in the transmission tube end connector (1280).

13. The thrombectomy device (100) according to claim 1, further comprising a thrombolytic system (2), wherein the thrombolytic system (2) comprises a syringe (1410), an infusion tube (1430), and a third check valve (1420); the infusion tube (1430) is connected to the syringe (1410); the third check valve (1420) is disposed in the infusion tube (1430); and the syringe (1410) injects a thrombolytic drug loaded therein to a thrombosis site through the infusion tube (1430), to soften or dissolve the thrombus at the thrombosis site.

## Patentansprüche

1. Thrombektomievorrichtung (100), **dadurch gekennzeichnet, dass** die Thrombektomievorrichtung Folgendes umfasst:
einen Thrombusentfernungskopf (4), der mindestens eine Öffnung aufweist, die konfiguriert ist, um zu ermöglichen, dass ein Thrombus in den Thrombusentfernungskopf (4) eindringen kann, und wobei der Thrombusentfernungskopf (4) mit einem Schaft (1340) verbunden ist;
ein Thrombusfragmentierungssystem (3), umfassend eine Betriebsanordnung, eine Getriebeanordnung (1200), einen Übertragungsschlauch (1260) und einen Thrombusfragmentierungsschneider (1270) umfasst, wobei die Betriebsanordnung den Übertragungsschlauch (1260) antreibt, um durch die Getriebeanordnung (1200) zu drehen, und ein distales Ende des Übertragungsschlauchs (1260) mit dem Thrombusfragmentierungsschneider (1270) verbunden ist; und
ein Thrombusabsaugsystem (1), umfassend ein Verbindungselement (1310), eine Absauganordnung (1320), einen Drainageschlauch (1370) und einen Flüssigkeitseinlassschlauch (1360), wobei ein Ende des Verbindungselements (1310) mit der Betriebsanordnung verbunden ist und ein weiteres Ende des Verbindungselements (1310) mit der Absauganordnung (1320) verbunden ist; wobei, wenn das Verbindungselement (1310) von der Betriebsanordnung angetrieben wird, es die Absauganordnung (1320) antreibt, um eine Druckkammer zu bilden; wobei die Druckkammer mit dem Drainageschlauch (1370) und dem Flüssigkeitseinlassschlauch (1360) verbunden ist; und der Flüssigkeitseinlassschlauch (1360) mit dem Schaft (1340) verbunden ist,
wobei die Absauganordnung (1320) eine Ansaugkammer (1321), einen Kolben (1326) und eine Kolbenstange (1325) aufweist; der Kolben (1326) über die Kolbenstange (1325) gestülpt und tangential zu einer Innenfläche der Ansaugkammer (1321) ist; ein von dem Kolben (1326) entferntes Ende der Kolbenstange (1325) mit dem Verbindungselement (1310) verbunden ist, und wobei ein Unterdruck in der Druckkammer erzeugt wird, wenn eine Wirkkraft auf die Betätigungsanordnung ausgeübt wird, und ein Überdruck in der Druckkammer erzeugt wird, wenn keine Wirkkraft auf die Betätigungsanordnung ausgeübt wird.

2. Thrombektomievorrichtung (100) nach Anspruch 1, wobei die Betätigungsanordnung einen Pressgriff (1110) und ein Übertragungselement (1210) umfasst; und das Übertragungselement (1210) eine Wirkkraft von dem Pressgriff (1110) auf die Getriebeanordnung (1200) überträgt.

3. Thrombektomievorrichtung (100) nach Anspruch 2, wobei der Pressgriff (1110) eine Schale (1130) und eine Torsionsfeder (1120), die in der Schale (1130) über einen Scharnierstift (1327) montiert ist, umfasst; wobei, wenn der Pressgriff (1110) von einer Presskraft befreit wird, der Pressgriff (1110) unter einer Wirkung der Torsionsfeder (1120) automatisch in einen natürlichen Zustand zurückkehrt.

4. Thrombektomievorrichtung (100) nach Anspruch 2, wobei das Übertragungselement (1210) eine bogenförmige Zahnstange ist.

5. Thrombektomievorrichtung (100) nach Anspruch 1, wobei die Betätigungsanordnung eine Wippe und eine Zahnradplatte (1231) umfasst und die Zahnradplatte (1231) mit der Zahnradeinheit (1200) in Eingriff ist.

6. Thrombektomievorrichtung (100) nach Anspruch 1, wobei die Getriebeanordnung (1200) ein erstes Kegelrad (1240) und ein zweites Kegelrad (1250) umfasst; das erste Kegelrad (1240) mit dem zweiten Kegelrad (1250) in Eingriff ist; das zweite Kegelrad (1250) an dem Übertragungsschlauch (1260) befestigt ist.

7. Thrombektomievorrichtung (100) nach Anspruch 6, wobei die Getriebeanordnung (1200) ferner ein erstes Zahnrad (1220), ein zweites Zahnrad (1230), eine Sperrklinke (1223) und ein drittes Zahnrad (1241) umfasst; die Betätigungsanordnung mit dem ersten Zahnrad (1220) in Eingriff ist; das erste Zahnrad (1220) sich die Sperrklinke (1223) mit dem zweiten Zahnrad (1230) teil; das zweite Zahnrad (1230) mit dem dritten Zahnrad (1241) in Eingriff ist; und das dritte Zahnrad (1241) koaxial mit dem ersten Kegelrad (1240) ist.

8. Thrombektomievorrichtung (100) nach einem der Ansprüche 1 bis 7, wobei die Absauganordnung (1320) ferner ein erstes Rückschlagventil (1324) und ein zweites Rückschlagventil (1324') umfasst, wobei das erste Rückschlagventil (1324) in dem Flüssigkeitseinlassschlauch (1360) angeordnet ist und das zweite Rückschlagventil (1324') in dem Drainageschlauch (1370) angeordnet ist.

9. Thrombektomievorrichtung (100) nach Anspruch 8, wobei das erste Rückschlagventil (1324) und das zweite Rückschlagventil (1324') in der gleichen Richtung angeordnet sind, sodass eines von dem ersten Rückschlagventil (1324) und dem zweiten Rückschlagventil (1324') offen ist, während das andere geschlossen ist.

10. Thrombektomievorrichtung (100) nach Anspruch 1, wobei das Verbindungselement (1310) von einer U-förmigen Struktur ist; eine untere Verbindungsstange (1311) der U-förmigen Struktur mit der Betätigungsanordnung verbunden ist und ein Öffnungsende der U-förmigen Struktur mit der Absauganordnung (1320) verbunden ist.

11. Thrombektomievorrichtung (100) nach Anspruch 1, wobei der Thrombusentfernungskopf (4) einen Hülsenanschluss (1350) umfasst; das Thrombusfragmentierungssystem (3) und das Thrombusabsaugsystem (1) durch den Hülsenanschluss (1350) mit dem Schaft (1340) verbunden sind; und sich der Übertragungsschlauch (1260) durch den Schaftverbinder (1350) erstreckt und in dem Schaft (1340) aufgenommen ist.

12. Thrombektomievorrichtung (100) nach Anspruch 1, wobei das Thrombusfragmentierungssystem (3) ferner einen Übertragungsschlauch-Endverbinder (1280) und ein einstellbares hämostatisches Ventil, das in dem Übertragungsschlauch-Endanschluss (1280) angeordnet ist, umfasst.

13. Thrombektomievorrichtung (100) nach Anspruch 1, ferner umfassend ein thrombolytisches System (2), wobei das thrombolytische System (2) eine Spritze (1410), einen Infusionsschlauch (1430) und ein drittes Rückschlagventil (1420) umfasst; wobei der Infusionsschlauch (1430) mit der Spritze (1410) verbunden ist; das dritte Rückschlagventil (1420) in dem Infusionsschlauch (1430) angeordnet ist; und die Spritze (1410) ein darin gefülltes thrombolytisches Arzneimittel durch den Infusionsschlauch (1430) zu einer Thrombosestelle injiziert, um den Thrombus an der Thrombosestelle aufzuweichen oder aufzulösen.

## Revendications

1. Dispositif de thrombectomie (100), **caractérisé par le fait que** le dispositif de thrombectomie comprend :
une tête d'élimination du thrombus (4) dotée d'au moins une ouverture configurée pour permettre au thrombus de pénétrer dans la tête d'élimination du thrombus (4), et la tête d'élimination du thrombus (4) étant reliée à une gaine (1340) ;
un système de fragmentation de thrombus (3) comprenant un ensemble de fonctionnement, un ensemble d'engrenages (1200), un tube de transmission (1260) et une fraise de fragmentation de thrombus (1270), dans lequel l'ensemble de fonctionnement entraîne le tube de transmission (1260) à tourner à travers l'ensemble d'engrenages (1200), et une extrémité distale du tube de transmission (1260) est reliée à la fraise de fragmentation de thrombus (1270) ; et
un système d'aspiration de thrombus (1) comprenant un élément de connexion (1310), un ensemble d'aspiration (1320), un tube de drainage (1370) et un tube d'entrée de liquide (1360), dans lequel une extrémité de l'élément de connexion (1310) est reliée à l'ensemble de fonctionnement, et une autre extrémité de l'élément de connexion (1310) est reliée à l'ensemble d'aspiration (1320) ; lorsqu'il est entraîné par l'ensemble de fonctionnement, l'élément de connexion (1310) entraîne l'ensemble d'aspiration (1320) pour former une chambre de pression ; la chambre de pression est reliée au tube de drainage (1370) et au tube d'entrée de liquide (1360) ; et le tube d'entrée de liquide (1360) est relié à la gaine (1340),
dans lequel l'ensemble d'aspiration (1320) comprend une chambre d'aspiration (1321), un piston (1326) et une tige de piston (1325) ; le piston (1326) s'insère dans la tige de piston (1325) et est tangent à une surface intérieure de la chambre d'aspiration (1321) ; une extrémité de la tige de piston (1325) éloignée du piston (1326) est reliée à l'élément de connexion (1310), et dans lequel une pression négative est générée dans la chambre de pression lorsqu'une force d'action est appliquée à l'ensemble de fonctionnement, et une pression positive est générée dans la chambre de pression lorsqu'aucune force d'action n'est appliquée à l'ensemble de fonctionnement.

2. Dispositif de thrombectomie (100) selon la revendication 1, dans lequel l'ensemble de fonctionnement comprend une poignée de pression (1110) et un élément de transmission (1210) ; et l'élément de transmission (1210) transmet une force d'action de la poignée de pression (1110) à l'ensemble d'engrenage (1200).

3. Dispositif de thrombectomie (100) selon la revendication 2, dans lequel la poignée de pression (1110) comprend une coque (1130) et un ressort de torsion (1120) monté dans la coque (1130) par l'intermédiaire d'une broche d'articulation (1327) ; lorsque la poignée de pression (1110) est libérée d'une force de pression, la poignée de pression (1110) revient automatiquement à un état naturel sous l'action du ressort de torsion (1120).

4. Dispositif de thrombectomie (100) selon la revendication 2, dans lequel l'élément de transmission (1210) est un rack arqué.

5. Dispositif de thrombectomie (100) selon la revendication 1, dans lequel l'ensemble de fonctionnement comprend une bascule et une plaque d'engrenage (1231), et la plaque d'engrenage (1231) est engrenée avec l'ensemble d'engrenage (1200).

6. Dispositif de thrombectomie (100) selon la revendication 1, dans lequel l'ensemble d'engrenages (1200) comprend un premier engrenage conique (1240) et un second engrenage conique (1250) ; le premier engrenage conique (1240) est engrené avec le second engrenage conique (1250) ; le second engrenage conique (1250) est fixé au tube de transmission (1260).

7. Dispositif de thrombectomie (100) selon la revendication 6, dans lequel l'ensemble d'engrenages (1200) comprend en outre un premier engrenage (1220), un deuxième engrenage (1230), un cliquet (1223) et un troisième engrenage (1241) ; l'ensemble de fonctionnement est engrené avec le premier engrenage (1220) ; le premier engrenage (1220) partage le cliquet (1223) avec le deuxième engrenage (1230) ; le deuxième engrenage (1230) est engrené avec le troisième engrenage (1241) ; et le troisième engrenage (1241) est coaxial avec le premier engrenage conique (1240).

8. Dispositif de thrombectomie (100) selon l'une quelconque des revendications 1 à 7, dans lequel l'ensemble d'aspiration (1320) comprend en outre un premier clapet anti-retour (1324) et un deuxième clapet anti-retour (1324') ; le premier clapet anti-retour (1324) est disposé dans le tube d'entrée du liquide (1360), et le deuxième clapet anti-retour (1324') est disposé dans le tube de drainage (1370).

9. Dispositif de thrombectomie (100) selon la revendication 8, dans lequel le premier clapet anti-retour (1324) et le second clapet anti-retour (1324') sont disposés dans la même direction de sorte que l'un du premier clapet anti-retour (1324) et du deuxième clapet anti-retour (1324') est ouvert tandis que l'autre est fermé.

10. Dispositif de thrombectomie (100) selon la revendication 1, dans lequel l'élément de connexion (1310) présente une structure en forme de U ; une tige de connexion inférieure (1311) de la structure en forme de U est connectée à l'ensemble de fonctionnement, et une extrémité d'ouverture de la structure en forme de U est connectée à l'ensemble d'aspiration (1320).

11. Dispositif de thrombectomie (100) selon la revendication 1, dans lequel la tête d'élimination du thrombus (4) comprend un connecteur de gaine (1350) ; le système de fragmentation du thrombus (3) et le système d'aspiration du thrombus (1) sont reliés à la gaine (1340) par l'intermédiaire du connecteur de gaine (1350) ; et le tube de transmission (1260) s'étend à travers le connecteur de gaine (1350) et est reçu dans la gaine (1340).

12. Dispositif de thrombectomie (100) selon la revendication 1, dans lequel le système de fragmentation du thrombus (3) comprend en outre un connecteur d'extrémité de tube de transmission (1280) et une valve hémostatique réglable disposée dans le connecteur d'extrémité de tube de transmission (1280).

13. Dispositif de thrombectomie (100) selon la revendication 1, comprenant en outre un système thrombolytique (2), dans lequel le système thrombolytique (2) comprend une seringue (1410), un tube de perfusion (1430), et un troisième clapet anti-retour (1420) ; le tube de perfusion (1430) est connecté à la seringue (1410) ; le troisième clapet anti-retour (1420) est disposé dans le tube de perfusion (1430) ; et la seringue (1410) injecte un médicament thrombolytique qu'elle contient dans un site de thrombose à travers le tube de perfusion (1430), afin de ramollir ou de dissoudre le thrombus au niveau du site de thrombose.
